# EUROPÄISCHE PATENTSCHRIFT

(11) **EP 1 776 077 B1**
(45) Veröffentlichungstag und Bekanntmachung des Hinweises auf die Patenterteilung: **10.11.2010**
(21) Anmeldenummer: 05764631.7
(22) Anmeldetag: 16.06.2005
(51) Int. Cl.: A61K 8/25, A61K 8/891, A61Q 5/02, A61K 8/46, A61K 8/44

(54) **VOLUMEN-HAARREINIGUNGSMITTEL**
VOLUME SHAMPOO
PRODUITS DE NETTOYAGE CAPILLAIRE VOLUMATEURS

(30) Priorität: 11.08.2004 DE 102004039158
(43) Veröffentlichungstag der Anmeldung: 25.04.2007
(73) Patentinhaber: Henkel AG & Co. KGaA, 40589 Düsseldorf (DE)
(72) Erfinder: GODDINGER, Dieter, 25336 Klein Nordende (DE); SCHRÖDER, Thomas, 22395 Hamburg (DE)
(86) Internationale Anmeldenummer: PCT/EP2005/006454
(87) Internationale Veröffentlichungsnummer: WO 2006/015649

(56) Entgegenhaltungen:
- EP-A- 0 155 806
- EP-A- 0 240 350
- EP-A- 0 287 773
- EP-A- 0 463 780
- DE-A1- 3 206 448
- US-A- 3 964 500
- US-A1- 2002 054 862
- US-A1- 2003 134 761

## Beschreibung

Die Erfindung betrifft Haarreinigungsmittel, welche dem mit ihnen behandelten Haar mehr Volumen und Fülle verleihen. Speziell betrifft die Erfindung Haarreinigungsmittel, enthaltend Silikon(e) und Metalloxid(e)sowie die Verwendung dieser Mittel zur Reinigung und/oder Pflege von Haut und Haar.

Die kosmetische Behandlung von Haut und Haaren ist ein wichtiger Bestandteil der menschlichen Körperpflege. So wird menschliches Haar heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen drei Typen von Haarfärbemitteln von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Weisen die im Verlauf der Farbausbildung gebildeten bzw. direkt eingesetzten Farbstoffe deutlich unterschiedliche Echtheiten (z. B. UV-Stabilität, Schweißechtheit, Waschechtheit etc.) auf, so kann es mit der Zeit zu einer erkennbaren und daher unerwünschten Farbverschiebung kommen. Dieses Phänomen tritt verstärkt auf, wenn die Frisur Haare oder Haarzonen unterschiedlichen Schädigungsgrades aufweist. Ein Beispiel dafür sind lange Haare, bei denen die lange Zeit allen möglichen Umwelteinflüssen ausgesetzten Haarspitzen in der Regel deutlich stärker geschädigt sind als die relativ frisch nachgewachsenen Haarzonen.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. In solchen Verfahren wird beispielsweise 5,6-Dihydroxyindolin als Farbstoffvorprodukt eingesetzt. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Nicht zuletzt durch die starke Beanspruchung der Haare, beispielsweise durch das Färben oder Dauerwellen als auch durch die Reinigung der Haare mit Shampoos und durch Umweltbelastungen, nimmt die Bedeutung von Pflegeprodukten mit möglichst langanhaltender Wirkung zu. Derartige Pflegemittel beeinflussen die natürliche Struktur und die Eigenschaften der Haare. So können anschließend an solche Behandlungen beispielsweise die Naß- und Trockenkämmbarkeit des Haares, der Halt und die Fülle des Haares optimiert sein oder die Haare vor erhöhtem Spliß geschützt sein.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung die Kämmbarkeit, der Halt und die Fülle der Haare verbessert und die Splißrate verringert.

Weiterhin wurden in jüngster Zeit sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate wirken im allgemeinen bevorzugt an der Haaroberfläche. So sind Wirkstoffe bekannt, welche dem Haar Glanz, Halt, Fülle, bessere Naß- oder Trockenkämmbarkeiten verleihen oder dem Spliß vorbeugen. Genauso bedeutend wie das äußere Erscheinungsbild der Haare ist jedoch der innere strukturelle Zusammenhalt der Haarfasern, der insbesondere bei oxidativen und reduktiven Prozessen wie Färbung und Dauerwellen stark beeinflußt werden kann.

Die bekannten Wirkstoffe können jedoch nicht alle Bedürfnisse in ausreichendem Maße abdecken. Es besteht daher weiterhin ein Bedarf nach Wirkstoffen bzw. Wirkstoffkombinationen für kosmetische Mittel mit guten pflegenden Eigenschaften und guter biologischer Abbaubarkeit. Insbesondere in farbstoff-und/oder elektrolythaltigen Formulierungen besteht Bedarf an zusätzlichen pflegenden Wirkstoffen, die sich problemlos in bekannte Formulierungen einarbeiten lassen.

Bei Mitteln zur Haarreinigung besteht unter anderem das Bedürfnis, den Haaren nach dem Trocknen Fülle und Volumen zu geben. Dieses Bedürfnis wird durch die Haarreinigungsmittel des Standes der Technik nur unzureichend befriedigt.

Es wurde nun gefunden, daß besonders vorteilhafte Ergebnisse erzielt werden können, wenn man Silikon(e) und Metalloxid(e) kombiniert. Beim Einsatz dieser Kombination kommt es zu überraschend guten Eigenschaften der behandelten Haare, insbesondere zu verbesserter Fülle, zu verbessertem Volumen und zu einer verbesserten Elastizität.

Ein erster Gegenstand der vorliegenden Erfindung ist daher ein Haarreinigungsmittel, enthaltend
a) mindestens ein Silikon der Formel I

   (CH₃)₃Si-[O-(CH₃)₂Si]ₓ-O-Si(CH₃)₃ (I),

   in der x für eine Zahl von 0 bis 10, vorzugsweise 0, 1 oder 2 steht und
b) mindestens ein Metalloxid,
   wobei das Gewichtsverhältnis von Silikon(en) zu Metalloxid(en) [a) zu b)] 7 : 1 bis 100 : 1, vorzugsweise 8 : 1 bis 50 : 1, besonders bevorzugt 9 : 1 bis 25 : 1 und insbesondere 10 : 1 bis 20 : 1 beträgt.
c) anionische, nichtionische, amphotere und/oder zwitterionische Tenside,
   wobei das Gewichtsverhältnis von Silikon(en) zu Metalloxid(en) [a] zu b)] 7 : 1 bis 100 : 1, vorzugsweise 8 : 1 bis 60 : 1, besonders bevorzugt 9 : 1 bis 25 : 1 und insbesondere 10 : 1 bis 20 : 1 beträgt und
   wobei die Partikelgröße der Metalloxide zwischen 2 und 100 nm, vorzugsweise zwischen 3 und 80 nm, besonders bevorzugt zwischen 4 und 60 nm und insbesondere zwischen 5 und 50 nm liegt.

Die erfindungsgemäßen Haareinigungsmittel enthalten als ersten Inhaltsstoff mindestens ein Silikon der vorstehenden Formel I. Diese Silikone werden nach der INCI-Nomenklatur als DIMETHICONE bezeichnet. Es werden im Rahmen der vorliegenden Erfindung als Silicon der Formel I die Verbindungen:

(CH₃)₃Si--O-Si(CH₃)₃

(CH₃)₃Si-O-(CH₃)₂Si-O-Si(CH₃)₃

(CH₃)₃Si-[O-(CH₃)₂Si]₂-O-Si(CH₃)₃

(CH₃)₃Si-[O-(CH₃)₂Si]₃-O-Si(CH₃)₃

(CH₃)₃Si-[O-(CH₃)₂Si]₄-O-Si(CH₃)₃

(CH₃)₃Si-[O-(CH₃)₂Si]₅-O-Si(CH₃)₃

(CH₃)₃Si-[O-(CH₃)₂Si]₆-O-Si(CH₃)₃

(CH₃)₃Si-[O-(CH₃)₂Si]₇-O-Si(CH₃)₃

(CH₃)₃Si-[O-(CH₃)₂Si]₈-O-Si(CH₃)₃

(CH₃)₃Si-[O-(CH₃)₂Si]₉-O-Si(CH₃)₃

(CH₃)₃Si-[O-(CH₃)₂Si]₁₀-O-Si(CH₃)₃

eingesetzt, wobei (CH₃)₃Si-O-Si(CH₃)₃, (CH₃)₃Si-O-(CH₃)₂Si-O-Si(CH₃)₃ und/oder (CH₃)₃Si-[O-(CH₃)₂Si]₂-O-Si(CH₃)₃ besonders bevorzugt sind.

Selbstverständlich können auch Mischungen der o.g. Silikone in den erfindungsgemäßen Mitteln enthalten sein. Das bzw. die Silikon(e) werden in erfindungsgemäßen Mitteln vorzugsweise in Mengen von bis zu 25 Gew.% eingesetzt Erfindungsgemäße Mittel, die bezogen auf das Gewicht des Mittels, 0,5 bis 20 Gew.%, vorzugsweise 1 bis 15Gew.%, besonders bevorzugt 2,5 bis 10 Gew.% und insbesondere 3 bis 7 Gew.% Silikon(e) enthalten, sind bevorzugte Ausführungsformen der vorliegenden Erfindung.

Bevorzugte erfindungsgemäß einsetzbare Silikone weisen bei 20°C Viskositäten von 0,2 bis 2 mm²s⁻¹ auf, wobei Silikone mit Viskosiäten von 0,5 bis 1 mm²s⁻¹ besonders bevorzugt sind.

Als zweiten wesentlichen Bestandteil enthalten die erfindungsgemäßen Mittel mindestens ein Metalloxid, wobei das Gewichtsverhältnis zwischen diesem Metalloxid und dem/den Silikon(en) der Formel (I) 1 : 7 bis 1 : 100, vorzugsweise 1 : 8 bis 1 : 50, besonders bevorzugt 1 : 9 bis 1 : 25 und insbesondere 1 : 10 bis 1 : 20, beträgt.

In bevorzugten erfindungsgemäßen Mitteln ist/sind das/die Metalloxid(e) bezogen auf das Gewicht des Mittels in Mengen von 0,01 bis 5 Gew.%, vorzugsweise von 0,1 bis 2,5 Gew.%, besonders bevorzugt von 0,25 bis 1,5 Gew.% und insbesondere von 0,3 bis 0,5 Gew.% enthalten.

Erfindungsgemäß einsetzbar sind alle Metalloxide, wobei die Oxide der Metalle der I. bis IV. Hauptgruppe des Periodensystems bevorzugt sind. Aber auch Oxide von Nebengruppenmetallen, wie beispielsweise Titandioxid, Zirkoniumdioxid, Vanadium(V)oxid, Ceroxid oder Zinkoxid können eingesetzt werden. Besonders bevorzugt sind die Oxide der Metalle der II., III. und IV. Hauptgruppe und unter diesen die Oxide von Magnesium, Calcium, Bor, Aluminium und Silicium. Besonders bevorzugte erfindungsgemäße Mittel sind **dadurch gekennzeichnet, daß** das Metalloxid ausgewählt ist aus Siliciumdioxid, Calciumoxid, Magnesiumoxid und Aluminiumoxid.

Das in den erfindungsgemäßen Mitteln enthaltene Metalloxid weist vorzugsweise eine bestimmte Stampfdichte auf. Bevorzugte erfindungsgemäße Mittel sind **dadurch gekennzeichnet, daß** das Metalloxid eine Stampfdichte gemäß DIN/ISO 787/11 von 10 - 400 g/l, vorzugsweise von 20 bis 300 g/l, besonders bevorzugt von 30 bis 200 g/l und insbesondere von 40 bis 120 g/l aufweist.

Vorteilhaft ist es auch, wenn das erfindungsgemäß in den Mitteln enthaltene Metalloxid eine bestimmte Oberfläche aufweist. Hier sind erfindungsgemäße Mittel bevorzugt, die dadurch gekennzeichnet sind, daß das Metalloxid eine BET-Oberfläche gemäß DIN 66131 von 50 bis 800 m²/g, vorzugsweise von 75 bis 600 m²/g, besonders bevorzugt von 100 bis 500 m²/g, und insbesondere von 130 bis 450 m²/g aufweist.

Die Metalloxide können in reiner Form oder chemisch modifiziert eingesetzt werden. Bevorzugte erfindungsgemäße Mittel sind **dadurch gekennzeichnet, daß** sie ein hydrophob modifiziertes Metalloxid enthalten. Die Hydrophobierung der Metalloxide kann dabei nach den gängigen Verfahren des Standes der Technik erfolgen, beispielsweise durch Behandlung des Metalloxids mit Silanen und/oder Enzymen und/oder Wachsen.

In einer besonders bevorzugten Ausführungsform der vorliegenden Erfindung werden Silikon(e) und Metalloxid(e) vor ihrer Einarbeitung in die erfindungsgemäßen Mittel miteinander vermischt und das Metalloxid dabei im Silikon dispergiert. Besonders bevorzugte erfindungsgemäße Mittel sind daher **dadurch gekennzeichnet, daß** sie eine Dispersion des Metalloxids b) im Silikon a) enthalten.

Solche Metalloxid/Silikon-Dispersionen enthalten vorzugsweise weniger als 25 Gew.% Metalloxid. Hier sind erfindungsgemäße Mittel bevorzugt, bei denen die Dispersion von Metalloxid in Silikon, bezogen auf das Gewicht der Dispersion, 2 bis 20 Gew.%, vorzugsweise 4 bis 15 Gew.%, besonders bevorzugt 5 bis 12 Gew.% und insbesondere 6 bis 10 Gew.% Metalloxid(e) enthält.

Selbstverständlich ist erfindungsgemäß auch beim Einsatz der vorstehend genannten Dispersionen das Gewichtsverhältnis zwischen Silikon und Metalloxid einzuhalten. Sollte dies im Einzelfall aus bestimmten Gründen mit der Dispersion allein nicht realisierbar sein, so sind die entsprechenden Mengen an Metalloxid bzw. Silikon auf anderem Wege als über die Dispersion in die erfindungsgemäßen Mittel einzuarbeiten. Insbesondere kann eine separate Dosierung eines Siilkons der Formel (I)nötig sein, wenn hochkonzentrierte Dispersionen von Metalloxiden in einem Silikon eingesetzt werden.

Zusätzlich zu den beiden Inhaltstoffen Silikon(en) und Metalloxid(en) können die erfindungsgemäßen Mittel weitere übliche Inhaltstoffe von Haarreinigungsmitteln enthalten.

Unter dem Begriff Tenside werden grenzflächenaktive Substanzen, die an Ober- und Grenzflächen Adsorptionsschichten bilden oder in Volumenphasen zu Mizellkolloiden oder lyotropen Mesophasen aggregieren können, verstanden. Man unterscheidet Aniontenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wäßriger Lösung stark hydratisiert sind.

Als anionische Tenside (E1) eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)_{X}-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- sulfatierte Hydroxyalkylpolyethylen- und/oder Hydroxyalkylenpropylenglykolether
- Sulfonate ungesättigter Fettsäuren mit 8 bis 24 C-Atomen und 1 bis 6 Doppelbindungen
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I),

   R¹(OCH₂CH₂)ₙ-O-P(O)(OX)-OR² (E1-I)

   in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- sulfatierte Fettsäurealkylenglykolester der Formel (E1-II) R⁷CO(AlkO)ₙSO₃M (E1-II)
   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht,
- Monoglyceridsulfate und Monoglyceridethersulfate der Formel (E1-III) 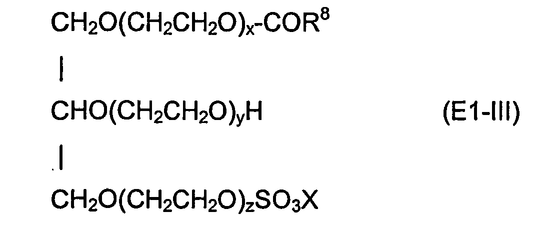 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht, ,
- Amidethercarbonsäuren,
- Kondensationsprodukte aus C₈ - C₃₀ - Fettalkoholen mit Proteinhydrolysaten und/oder Aminosäuren und deren Derivaten,welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside (E2) werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden (E3) werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside (E4) enthalten als hydrophile Gruppe z.B. eine Polyolgruppe, eine Polyalkylenglykolethergruppe oder eine Kombination aus Polyol-und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.
   Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III),

   R⁵CO-NR⁶-[Z] (E4-III)

   in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R⁷CO-NR⁸-CH₂-(CHOH)₄-CH₂OH (E4-IV)

Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "OxoAlkohole" als Ausgangsstoffe überwiegen Verbindungen mit einer ungeraden Anzahl von Kohlenstoffatomen in der Alkylkette.

Weiterhin sind ganz besonders bevorzugte nichtionische Tenside die Zuckertenside. Diese können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Erfindungsgemäß einsetzbar sind auch kationische Tenside vom Typ der quarternären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bevorzugt einsetzbar sind erfindungsgemäß QAV mit Behenylresten, insbesondere die unter der Bezeichnung Behentrimoniumchlorid bzw. -bromid (Docosanyltrimethylammonium Chlorid bzw. -Bromid) bekannten Substanzen. Andere bevorzugte QAV weisen mindestens zwei Behenylreste auf, wobei QAV, welche zwei Behenylreste an einem Imidazoliniumrückgrat besonders bevorzugt sind. Kommerziell erhältlich sind diese Substanzen beispielsweise unter den Bezeichnungen Genamin^{®} KDMP (Clariant) und Crodazosoft^{®} DBQ (Crodauza).

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Die Tenside (E), d.h. die Summe aller Tenside, werden vorzugsweise in Mengen von 0,1 - 45 Gew.%, bevorzugt 0,5 - 30 Gew.% und ganz besonders bevorzugt von 0,5 - 25 Gew.%, bezogen auf das gesamte erfindungsgemäße Mittel, eingesetzt.

Anionische, nichtionische, zwitterionische und/oder amphotere Tenside sowie deren Mischungen sind erfindungsgemäß bevorzugt.

Die erfindungsgemäßen Mittel können darüber hinaus vorzugsweise mindestens eine Polyhydroxyverbindung mit mindestens 2 OH-Gruppen enthalten. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 1, 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden.

Wichtige Vertreter von Polyhydroxyverbindungen mit 2 OH-Gruppen sind auch die Polyethylen- und Polypropylenglycole.

Unter den Polymhydroxyverbindungen mit 3 OH-Gruppen hat das Gylcerin eine herausragende Bedeutung.

Zusammenfassend sind erfindungsgemäße Mittel bevorzugt, die zusätzlich (eine) Polyhydroxyverbindung(en) enthalten, die vorzugsweise ausgewählt ist/sind aus Ethylenglycol, Propylenglycol, Polyethylenglycol, Polypropylenglycol, Glycerin, Pentaerythrit, Sorbit, Mannit, Xylit und ihren Mischungen.

Unabhängig vom Typ der eingesetzten Polyhydroxyverbindung mit mindestens 2 OH-Gruppen sind erfindungsgemäße Mittel bevorzugt, die, bezogen auf das Gewicht des Mittels, 0,01 bis 5 Gew.%, vorzugsweise 0,1 bis 4 Gew.%, besonders bevorzugt 0,15 bis 3,5 Gew.% und insbesondere 0,3 bis 2,5 Gew.% Polyhydroxyverbindung(en) enthalten.

Mit besonderem Vorzug können die erfindungsgemäßen Mittel zusätzlich Polyethylenglycolether der Formel (II)

H(CH₂)ₖ(OCH₂CH₂)ₙOH (II)

enthalten, worin k eine Zahl zwischen 1 und 18 unter besonderer Bevorzugung der Werte 0, 10, 12, 16 und 18 und n eine Zahl zwischen 2 und 20 unter besonderer Bevorzugung der Werte 2, 4, 5, 6, 7, 8, 9, 10, 12 und 14 bedeutet. Bevorzugt sind unter diesen die Alkylderivate des Diethylenglycols, des Triethylenglycols, des Tetraethylenglycols, des Pentathylenglycols, des Hexaethylenglycols, des Heptaethylenglycols, des Octaethylenglycols, des Nonaethylenglycols, des Decaethylenglycols, des Dodecaethylenglycols und des Tetradecaethylenglycols sowie die Alkylderivate des Dipropylenglycols, des Tripropylenglycols, des Tetrapropylenglycols, des Pentapropylenglycols, des Hexapropylenglycols, des Heptapropylenglycols, des Octapropylenglycols, des Nonapropylenglycols, des Decapropylenglycols, des Dodecapropylenglycols und des Tetradecapropyolenglycols, wobei unter diesen die Methyl-, Ehyl-, Propyl-, n-Butyl, n-Pentyl, n-Hexyl-, n-Heptyl-, n-Octyl-, n-Nonyl, n-Decyl-, n-Undecyl-, n-Dodecyl- und n-Tetradecyl-Derivate bevorzugt sind.

Es hat sich gezeigt, daß Mischungen "kurzkettiger" Polyalkylenglycolether mit solchen "langkettiger" Polyalkylenglycolether Vorteile besitzen. "Kurz- bzw. langkettig" bezieht sich in diesem Zusammenhang auf den Polymerisationsgrad des Polyalkylenglycols. Besonders bevorzugt sind Mischungen von Polyalkylenglycolethern mit einem Oligomerisierungsgrad von 5 oder weniger mit Polyalkylenglycolethern mit einem Oligomerisierungsgrad von 7 oder mehr. Bevorzugt sind Mischungen von Alkylderivaten des Diethylenglycols, des Triethylenglycols, des Tetraethylenglycols, des Pentathylenglycols, des Dipropylenglycols, des Tripropylenglycols, des Tetrapropylenglycols oder des Pentapropylenglycols mit Alkylderivaten des Hexaethylenglycols, des Heptaethylenglycols, des Octaethylenglycols, des Nonaethylenglycols, des Decaethylenglycols, des Dodecaethylenglycols, des Hexapropylenglycols, des Heptapropylenglycols, des Octapropylenglycols, des Nonapropylenglycols, des Decapropylenglycols, des Dodecapropylenglycols oder des Tetradecapropyolenglycols, wobei in beiden Fällen die n-Octyl-, n-Decyl-, n-Dodecyl- und n-Tetradecyl-Derivate bevorzugt sind.

Besonders bevorzugte erfindungsgemäße Mittel sind **dadurch gekennzeichnet, daß** es mindestens einen Polyalkylenglycolether (IIa) der Formel (II), in der n für die Zahlen 2, 3, 4 oder 5 steht und mindestens einen Polyalkylenglycolether (IIb)der Formel (II) enthält, in der n für die Zahlen 10, 12, 14 oder 16 steht, wobei das Gewichtsverhältnis (II b) zu (II a) 10 : 1 bis 1 : 10, vorzugsweise 7,5 : 1 bis 1 : 5 und insbesondere 5 : 1 bis 1 : 1 beträgt.

Zusätzlich zu den genannten Stoffen können die erfindungsgemäßen Mittel weitere Inhaltsstoffe enthalten. Mit besonderem Vorzug sind dies beispielsweise Vitamine, Provitamine oder Vitaminvorstufen, so daß erfindungsgemäß bevorzugte Mittel dadurch gekennzeichnet sind, daß sie zusätzlich mindestens einen Stoff aus der Gruppe der Vitamine, Provitamine und Vitaminvorstufen sowie deren Derivate enthalten, wobei Vitamine, Pro-Vitamine und Vitaminvorstufen bevorzugt sind, die den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Mittel Vitamine, Provitamine und Vitaminvorstufen aus den Gruppen A, B, E und H. Panthenol, Pantolacton, Pyridoxin und seine Derivate sowie Nicotinsäureamid und Biotin sind besonders bevorzugt.

Mit besonderem Vorzug enthalten die erfindungsgemäßen Mittel zusätzlich ein aminofunktionelles Silikon der Formel (III)

R'ₐG₃₋ₐ-Si(OSiG ₂)ₙ-(OSiG _{b}R'_{2-b})ₘ-O-SiG₃₋ₐ-R'ₐ (III),

enthält, worin bedeutet:
- G ist-H, eine Phenylgruppe, -OH, -O-CH₃, -CH₃, -CH₂CH₃, - CH₂CH₂CH₃, -CH(CH₃)₂, -CH₂CH₂CH₂H₃, -CH₂CH(CH₃)₂, - CH(CH₃)CH₂CH₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ○ -N(R")-CH₂-CH ₂- N(R")₂
   ○ -N(R")₂
   ○ -N⁺(R")₃A⁻
   ○ -N⁺H(R")₂ A⁻
   ○ -N⁺H₂(R")A⁻
   ○ -N(R")-CH₂-CH₂-N⁺R"H₂A⁻,
wobei jedes R" für gleiche oder verschiedene Reste aus der Gruppe -H, -Phenyl, -Benzyl, der C₁₋₂₀-Alkylreste, vorzugsweise -CH₃, -CH₂CH₃, -CH₂CH₂CH₃, - CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Besonders bevorzugte erfindungsgemäße Mittel sind **dadurch gekennzeichnet, daß** sie ein aminofunktionelles Silikon der Formel (IV) 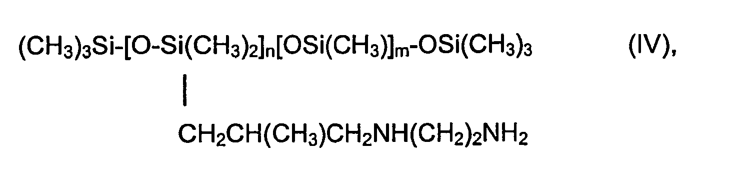 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Besonders bevorzugt sind auch erfindungsgemäße Mittel, die dadurch gekennzeichnet sind, daß sie ein aminofunktionelles Silikon der Formel (V) 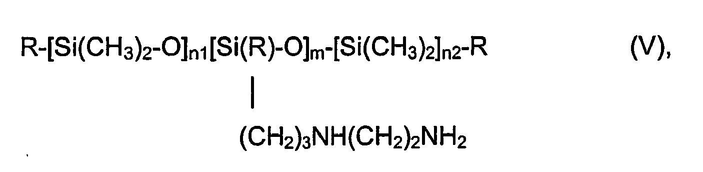 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, bei denen das aminofunktionelle Silikon eine Aminzahl oberhalb von 0,25 meq/g, vorzugsweise oberhalb von 0,3 meq/g und insbesondere oberhalb von 0,4 meq/g aufweist. Die Aminzahl steht dabei für die Milli-Äquivalente Amin pro Gramm des aminofunktioinelen Silicons. Sie kann durch Titration ermittelt und auch in der Einheit mg KOH/g angegeben werden.

Erfindungsgemäß bevorzugte Mittel enthalten, bezogen auf das Gewicht des Mittels, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e).

In einer bevorzugten Ausführungsform der Erfindung kann die Wirkung der erfindungsgemäßen Mittel durch Fettstoffe (D) weiter gesteigert werden. Unter Fettstoffen sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Als Fettsäuren (D1) können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1 - 5 Gew.% sein können.

Als Fettalkohole (D2) können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 - 20 Gew.-% eingesetzt.

Als natürliche oder synthetische Wachse (D3) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Zu den natürlichen und synthetischen kosmetischen Ölkörpern (D4), welche die Wirkung des erfindungsgemäßen Wirkstoffkomplexes (A) steigern können, sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methylpentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Ritanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-I), 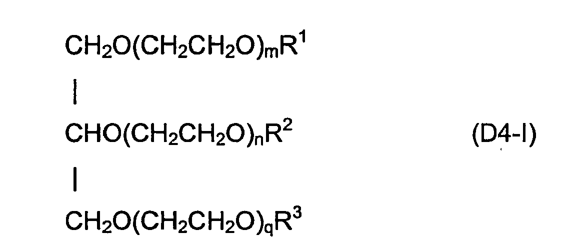 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, daß mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Mitteln beträgt üblicherweise 0,1 - 30 Gew.%, bezogen auf das gesamte Mittel, bevorzugt 0,1 - 20 Gew.-%, und insbesondere 0,1 - 15 Gew.-%.

Die Gesamtmenge an Öl- und Fettkomponenten in den erfindungsgemäßen Mitteln beträgt üblicherweise 0,5 - 75 Gew.-%, bezogen auf das gesamte Mittel. Mengen von 0,5 - 35 Gew.-% sind erfindungsgemäß bevorzugt.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®} 68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Als weiterhin vorteilhaft hat es sich gezeigt, wenn Polymere (G) in den erfindungsgemäßen Mitteln enthalten sind. In einer bevorzugten Ausführungsform werden den erfindungsgemäß verwendeten Mitteln daher Polymere zugesetzt, wobei sich sowohl kationische, anionische, amphotere als auch nichtionische Polymere als wirksam erwiesen haben.

Erfindungsgemäß einsetzbar sind mit besonderem Vorteil auch kationische Polymere. Unter kationischen Polymerensind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 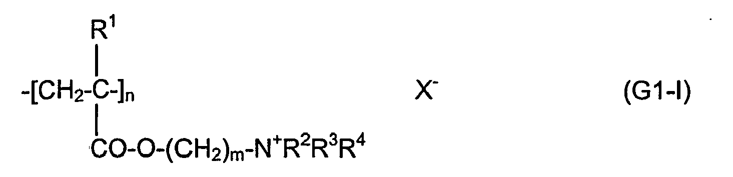 in der R¹= -H oder -CH₃ ist, R², R³ und R⁴ unabhängig voneinander ausgewählt sind aus C1-4-Alkyl-, Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (Ethnichem) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- polymere Dimethyldiallylammoniumsalze und deren Copolymere mit Estern und Amiden von Acrylsäure und Methacrylsäure. Die unter den Bezeichnungen Merqua^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoff^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere in den erfindungsgemäßen Mitteln einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Weiterhin sind kationiserte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und - derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und - derivate auf pflanzlicher Basis.

Die kationischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Weiterhin können als Polymere amphotere Polymere (G3) verwendet werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻ - oder -SO₃⁻-Gruppen enthalten, und solche Polymere zusammengefaßt, die - COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Bevorzugt eingesetzte amphotere Polymere sind solche Polymerisate, die sich im wesentlichen zusammensetzen aus
(a) Monomeren mit quartären Ammoniumgruppen der allgemeinen Formel (G3-I),

   R¹-CH=CR²-CO-Z-(CₙH₂ₙ)-N⁽⁺⁾R³R⁴R⁵ A⁽⁻⁾ (G3-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (G3-II),

   R⁶-CH=CR⁷-COOH (G3-II)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethylammoniumchlorid ist ein besonders bevorzugtes Monomeres (a). Als Monomeres (b) für die genannten Polymerisate wird bevorzugt Acrylsäure verwendet.

Die erfindungsgemäßen Mittel können in einer weiteren Ausführungsform nichtionogene Polymere (G4) enthalten.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Es ist erfindungsgemäß auch möglich, daß die verwendeten Zubereitungen mehrere, insbesondere zwei verschiedene Polymere gleicher Ladung und/oder jeweils ein ionisches und ein amphoteres und/oder nicht ionisches Polymer enthalten.

Die Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Weiterhin kann in einer bevorzugten Ausführungsform der Erfindung ein erfindungsgemäßes Mittel auch UV - Filter (I) enthalten. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul^{®}M 40, Uvasorb^{®}MET, Neo Heliopan^{®}BB, Eusolex^{®}4360), 2-Phenylbenzimidazol-5-sulfonsäure und deren Kalium-, Natrium- und Triethanolaminsalze (Phenylbenzimidazole sulfonic acid; Parsol^{®}HS; Neo Heliopan^{®}Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol^{®}1789, Eusolex^{®}9020), α-(2-Oxoborn-3-yliden)-toluol-4-sulfonsäure und deren Salze, ethoxylierte 4-Aminobenzoesäure-ethylester (PEG-25 PABA; Uvinul^{®}P 25), 4-Dimethylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}O18), 4-Methoxyzimtsäure-isopentylester (Isoamyl p-Methoxycinnamate; Neo Heliopan^{®}E 1000), 4-Methoxyzimtsäure-2-ethylhexyl-ester (Octyl Methoxycinnamate; Parsol^{®}MCX, Escalol^{®}557, Neo Heliopan^{®}AV), 2-Hydroxy-4-methoxybenzophenon-5-sulfonsäure und deren Natriumsalz (Benzophenone-4; Uvinul^{®}MS 40; Uvasorb^{®}S 5), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahydroxybenzophenon (Benzophenone-2; Uvinul^{®}D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, daß bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Diese UV-Filter weisen die allgemeine Struktur U - Q auf.

Der Strukturteil U steht dabei für eine UV-Strahlen absorbierende Gruppe. Diese Gruppe kann sich im Prinzip von den bekannten, im Kosmetikbereich einsetzbaren, oben genannten UV-Filtern ableiten, in dem eine Gruppe, in der Regel ein Wasserstoffatom, des UV-Filters durch eine kationische Gruppe Q, insbesondere mit einer quartären Aminofunktion, ersetzt wird.

Verbindungen, von denen sich der Strukturteil U ableiten kann, sind beispielsweise
- substituierte Benzophenone,
- p-Aminobenzoesäureester,
- Diphenylacrylsäureester,
- Zimtsäureester,
- Salicylsäureester,
- Benzimidazole und
- o-Aminobenzoesäureester.

Strukturteile U, die sich vom Zimtsäureamid oder vom N,N-Dimethylaminobenzoesäureamid ableiten, sind erfindungsgemäß bevorzugt.

Die Strukturteile U können prinzipiell so gewählt werden, daß das Absorptionsmaximum der UV-Filter sowohl im UVA(315-400 nm)-, als auch im UVB(280-315nm)- oder im UVC(<280 nm)-Bereich liegen kann. UV-Filter mit einem Absorptionsmaximum im UVB-Bereich, insbesondere im Bereich von etwa 280 bis etwa 300 nm, sind besonders bevorzugt.

Weiterhin wird der Strukturteil U, auch in Abhängigkeit von Strukturteil Q, bevorzugt so gewählt, daß der molare Extinktionskoeffizient des UV-Filters am Absorptionsmaximum oberhalb von 15 000, insbesondere oberhalb von 20000, liegt.

Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so daß der Strukturteil U einen der vier Substituenten des positiv geladenen Stickstoffatomes darstellt. Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.

Vorteilhafterweise hat die Gruppe Q die allgemeine Struktur -(CH₂)_{X}-N⁺R¹R²R³ X⁻ , in der x steht für eine ganze Zahl von 1 bis 4, R¹ und R² unabhängig voneinander stehen für C₁₋₄-Alkylgruppen, R³ steht für eine C₁₋₂₂-Alkylgruppe oder eine Benzylgruppe und X⁻ für ein physiologisch verträgliches Anion. Im Rahmen dieser allgemeinen Struktur steht x bevorzugt für die die Zahl 3, R¹ und R² jeweils für eine Methylgruppe und R³ entweder für eine Methylgruppe oder eine gesättigte oder ungesättigte, lineare oder verzweigte Kohlenwasserstoffkette mit 8 bis 22, insbesondere 10 bis 18, Kohlenstoffatomen.

Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat.

Zwei bevorzugte UV-Filter mit kationischen Gruppen sind die als Handelsprodukte erhältlichen Verbindungen Zimtsäureamidopropyltrimethylammoniumchlorid (Incroquat^{®}UV-283) und Dodecyldimethylaminobenzamidopropyl-dimethylammoniumtosylat (Escalol^{®} HP 610).

Selbstverständlich umfaßt die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter (I) sind in den erfindungsgemäß verwendeten Mitteln üblicherweise in Mengen 0,1-5 Gew.-%, bezogen auf das gesamte Mittel, enthalten. Mengen von 0,4-2,5 Gew.-% sind bevorzugt.

Die erfindungsgemäßen Mittel können weiterhin eine 2-Pyrrolidinon-5-carbonsäure und deren Derivate (J) enthalten. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.%, bezogen auf das gesamte Mittel, besonders bevorzugt 0,1 bis 5, und insbesondere 0,1 bis 3 Gew.%.

Schließlich können die erfindungsgemäßen Mittel auch Pflanzenextrakte (L) enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Zusätzlich kann es sich als vorteilhaft erweisen, wenn in den erfindungsgemäßen Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Vorteilhaft im Sinne der Erfindung können zusätzlich kurzkettige Carbonsäuren (N) den Wirkstoffkomplex (A) unterstützen. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäßen Carbonsäuren können selbstverständlich entlang der Kohlenstoffkette oder des Ringgerüstes substituiert sein. Zu den Substituenten der erfindungsgemäßen Carbonsäuren sind beispielsweise zu zählen C1-C8-Alkyl-, C2-C8-Alkenyl-, Aryl-, Aralkyl- und Aralkenyl-, Hydroxymethyl-, C2-C8-Hydroxyalkyl-,C2-C8-Hydroxyalkenyl-, Aminomethyl-, C2-C8-Aminoalkyl-, Cyano-, Formyl-, Oxo-, Thioxo-, Hydroxy-, Mercapto-, Amino-, Carboxy- oder Iminogruppen. Bevorzugte Substituenten sind C1-C8-Alkyl-, Hydroxymethyl-, Hydroxy-, Amino- und Carboxygruppen. Besonders bevorzugt sind Substituenten in □ - Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Als Beispiele für erfindungsgemäße Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxyphthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I),

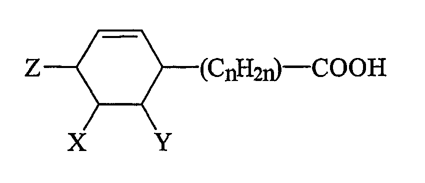

### (N-I)

in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Dicarbonsäuren der Formel (N-I) sind in der Literatur bekannt.

Die Dicarbonsäuren der Formel (N-I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuß vorliegt. Diese Isomerengemische können erfindungsgemäß ebenso wie die reinen Verbindungen eingesetzt werden.

Erfindungsgemäß einsetzbar neben den bevorzugten Dicarbonsäuren gemäß Formel (N-I) sind auch solche Dicarbonsäuren, die sich von den Verbindungen gemäß Formel (N-I) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid^{®} 1550 und Westvaco Diacid^{®} 1595 (Hersteller: Westvaco) erhältlich.

Neben den zuvor beispielhaft aufgeführten erfindungsgemäßen kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und - hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren gemeinsam mit dem Wirkstoff (A) einzusetzen. Hierbei hat sich gezeigt, daß neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C12-C15-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

Die erfindungsgemäßen Mittel können zusätzlich Proteinhydrolysate und deren Derivate (P) enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton. Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda) oder Crotein^{®} (Croda) vertrieben.

Selbstverständlich umfaßt die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten (P) einzusetzen.

Die Proteinhydrolysate (P) sind in den Mitteln in Konzentrationen von 0,01 Gew.% bis zu 20 Gew.%, vorzugsweise von 0,05 Gew.% bis zu 15 Gew.% und ganz besonders bevorzugt in Mengen von 0,05 Gew.% bis zu 5 Gew.% enthalten.

Die erfindungsgemäßen Mittel können als Shampoos, Konditioniermittel, Haarsprays usw. formuliert werden, wobei die Mittel fest, flüssig oder sprühbar konfektioniert sein können. Für Anwendungen, bei denen das Haar gleichzeitig oder später gefärbt oder getönt werden soll, können die Mittel auch als Haarfärbemittel oder Haartönung formuliert werden.

Hinsichtlich der Art, gemäß welcher der erfindungsgemäße Wirkstoffkomplex auf die keratinische Faser, insbesondere das menschliche Haar, aufgebracht wird, bestehen keine prinzipiellen Einschränkungen. Als Konfektionierung dieser Zubereitungen sind beispielsweise Cremes, Lotionen, Lösungen, Wässer, Emulsionen wie W/O-, O/W-, PIT-Emulsionen (Emulsionen nach der Lehre der Phaseninversion, PIT genannt), Mikroemulsionen und multiple Emulsionen, Gele, Sprays, Aerosole und Schaumaerosole geeignet. Der pH-Wert dieser Zubereitungen kann prinzipiell bei Werten von 2 - 11 liegen. Er liegt bevorzugt zwischen 5 und 11, wobei Werte von 6 bis 10 besonders bevorzugt sind. Zur Einstellung dieses pH-Wertes kann praktisch jede für kosmetische Zwecke verwendbare Säure oder Base verwendet werden. Bevorzugte Basen sind Ammoniak, Alkalihydroxide, Monoethanolamin, Triethanolamin sowie N,N,N',N'-Tetrakis-(2-hydroxypropyl)-ethylendiamin.

Auf dem Haar verbleibende Zubereitungen haben sich als wirksam erwiesen und können daher bevorzugte Ausführungsformen der erfindungsgemäßen Lehre darstellen. Unter auf dem Haar verbleibend werden erfindungsgemäß solche Zubereitungen verstanden, die nicht im Rahmen der Behandlung nach einem Zeitraum von wenigen Sekunden bis zu einer Stunde mit Hilfe von Wasser oder einer wäßrigen Lösung wieder aus dem Haar ausgespült werden. Vielmehr verbleiben die Zubereitungen bis zur nächsten Haarwäsche, d.h. in der Regel mehr als 12 Stunden, auf dem Haar.

Gemäß einer zweiten bevorzugten Ausführungsform werden diese Zubereitungen als Haarkur oder Haar-Conditioner formuliert. Die erfindungsgemäßen Zubereitungen gemäß dieser Ausführungsform können nach Ablauf dieser Einwirkzeit mit Wasser oder einem zumindest überwiegend wasserhaltigen Mittel ausgespült werden; sie können jedoch, wie oben ausgeführt, auf dem Haar belassen werden. Dabei kann es bevorzugt sein, die erfindungsgemäße Zubereitung vor der Anwendung eines reinigenden Mittels, eines Wellmittels oder anderen Haarbehandlungsmitteln auf das Haar aufzubringen. In diesem Falle dient die erfindungsgemäße Zubereitung als Strukturschutz für die nachfolgenden Anwendungen.

Gemäß weiteren bevorzugten Ausführungsformen kann es sich bei den erfindungsgemäßen Mitteln aber beispielsweise auch um reinigende Mittel wie Shampoos, pflegende Mittel wie Spülungen, festigende Mittel wie Haarfestiger, Schaumfestiger, Styling Gels und Fönwellen, dauerhafte Verformungsmittel wie Dauerwell- und Fixiermittel sowie insbesondere im Rahmen eines Dauerwellverfahrens oder Färbeverfahrens eingesetzte Vorbehandlungsmittel oder Nachspülungen handeln.

Neben dem erfindungsgemäß zwingend erforderlichen Wirkstoffkomplex (A) und den weiteren, oben genannten bevorzugten Komponenten können diese Zubereitungen prinzipiell alle weiteren, dem Fachmann für solche kosmetischen Mittel bekannten Komponenten enthalten.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- symmetrische und unsymmetrische, lineare und verzweigte Dialkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether und Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether,
- Fettalkohole, insbesondere lineare und/oder gesättigte Fettalkohole mit 8 bis 30 C-Atomen,
- Monoester von C8 bis C30 - Fettsäuren mit Alkoholen mit 6 bis 24 C-Atomen,
- faserstrukturverbessernde Wirkstoffe, insbesondere Mono-, Di- und Oligosaccharide, wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie
- Phospholipide, beispielsweise Sojalecithin, Ei-Lecithin und Kephaline,
- quaternierte Amine wie Methyl-1-alkylamidoethyl-2-alkylimidazolinium-methosulfat,
- Entschäumer wie Silikone,
- Farbstoffe zum Anfärben des Mittels,
- Antischuppenwirkstoffe wie Piroctone Olamine, Zink Omadine und Climbazol,
- Wirkstoffe wie Allantoin und Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Reduktionsmittel wie z. B. Thioglykolsäure und deren Derivate, Thiomilchsäure, Cysteamin, Thioäpfelsäure und α-Mercaptoethansulfonsäure,
- Treibmittel wie Propan-Butan-Gemische, N₂O, Dimethylether, CO₂ und Luft,
- Antioxidantien.

In einer weiteren Ausführungsform der erfindungsgemäßen Lehre kann es bevorzugt sein, dem erfindungsgemäßen Haarreinigungsmittel in Form eines farberhaltenden oder farbauffrischenden Mittels bereitzustellen, das bedeutet, die erfindungsgemäßen Mittel mit Farbstoffen und/oder Farbstoffvorprodukten einzusetzen.

Als solche können Oxidationsfarbstoffvorprodukte vom Entwickler- (B1) und Kuppler-Typ ( B2), natürliche und synthetische direktziehende Farbstoffe (C) und Vorstufen naturanaloger Farbstoffe, wie Indol- und Indolin-Derivate, sowie Mischungen von Vertretern einer oder mehrerer dieser Gruppen eingesetzt werden.

Als Oxidationsfarbstoffvorprodukte vom Entwickler-Typ (B1) werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Geeignete Entwicklerkomponenten sind beispielsweise p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diamino-phenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 741 bzw. WO 94/08970 wie z. B. 4,5-Diamino-1-(2'-hydroxyethyl)-pyrazol. Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin.

Als Oxidationsfarbstoffvorprodukte vom Kuppler-Typ (B2) werden in der Regel m-Phenylendiaminderivate, Naphthole, Resorcin und Resorcinderivate, Pyrazolone und m-Aminophenolderivate verwendet. Beispiele für solche Kupplerkomponenten sind m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol, o-Aminophenol und dessen Derivate, m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol, o-Diaminobenzol und dessen Derivate wie beispielsweise 3,4-Diaminobenzoesäure und 2,3-Diamino-1-methylbenzol, Di- beziehungsweise Trihydroxybenzolderivate wie beispielsweise Resorcin, Resorcin-monomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol, Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin, Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin, Morpholinderivate wie beispielsweise 6-Hydroxybenzomorpholin und 6-Amino-benzomorpholin, Chinoxalinderivate wie beispielsweise 6-Methyl-1,2,3,4-tetrahydrochinoxalin, Pyrazolderivate wie beispielsweise 1-Phenyl-3-methylpyrazol-5-on, Indolderivate wie beispielsweise 4-Hydroxyindol, 6-Hydroxyindol und 7-Hydroxyindol, Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders geeignete Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Direktziehende Farbstoffe sind üblicherweise Nitrophenylendiamine, Nitroaminophenole, Azofarbstoffe, Anthrachinone oder Indophenole. Besonders geeignete direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(3-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

In der Natur vorkommende direktziehende Farbstoffe sind beispielsweise Henna rot, Henna neutral, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Als Vorstufen naturanaloger Farbstoffe werden beispielsweise Indole und Indoline sowie deren physiologisch verträgliche Salze verwendet. Bevorzugt werden solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders vorteilhafte Eigenschaften haben 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin sowie 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin sowie N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Sowohl die Oxidationsfarbstoffvorprodukte als auch die direktziehenden Farbstoffe und die Vorstufen naturanaloger Farbstoffe sind in den erfindungsgemäßen Mitteln bevorzugt in Mengen von 0,01 bis 20 Gew.-%, vorzugsweise 0,1 bis 5 Gew.-%, jeweils bezogen auf das gesamte Mittel, enthalten.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Erfolgt die Ausbildung der eigentlichen Haarfarben im Rahmen eines oxidativen Prozesses, so können übliche Oxidationsmittel, wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel, oder auch Enzyme verwendet werden, die Elektronen aus geeigneten Entwicklerkomponenten (Reduktionsmittel) auf Luftsauerstoff übertragen. Bevorzugt sind dabei Oxidasen wie Tyrosinase, Ascorbatoxidase und Laccase aber auch Glucoseoxidase, Uricase oder Pyruvatoxidase. Weiterhin sei das Vorgehen genannt, die Wirkung geringer Mengen (z. B. 1 % und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Weiterhin kann die Ausbildung der Färbung dadurch unterstützt und gesteigert werden, daß dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Ein weiterer Gegenstand der vorliegenden Erfindung ist ein Verfahren zur Herstellung von Haarreinigungsmitteln, bei dem mindestens ein Metalloxid in mindestens einem Silikon der Formel I

(CH₃)₃Si-[O-(CH₃)₂Si]ₓ-O-Si(CH₃)₃ (I),

in der x für eine Zahl von 0 bis 10, vorzugsweise 0, 1 oder 2 steht, dispergiert und diese Dispersion mit weiteren Inhaltstoffen von Haarreinigungsmitteln vermischt wird.

Bezüglich bevorzugter Mengen an Silikon(en) und Metalloxid(en), sowohl in der Dispersion als auch im fertigen Haarreinigungsmittel, bezüglich bevorzugter Gewichtsverhältnisse zwischen Silikon(en) und Metalloxid(en) sowie bezüglich weiterer bevorzugter Inhaltsstoffe gilt mutatis mutandis das zu den erfindungsgemäßen Mitteln Gesagte.

Weitere Gegenstände der vorliegenden Erfindung sind die Verwendung einer erfindungsgemäßen Zubereitung zur Reinigung von Haut und Haar sowie die Verwendung einer erfindungsgemäßen Zubereitung zur Fülle- und Volumenerhöhung von keratinischen Fasern, insbesondere menschlichen Haaren.

Ein weiterer Gegenstand der vorliegenden Erfindung ist ein Verfahren zur Behandlung von Haut oder Haar, bei dem eine erfindungsgemäße Zubereitung auf die Haut und/oder das Haar aufgetragen wird, wobei die Zubereitung nach einer Einwirkzeit von 0 bis 45 Minuten wieder ausgespült wird.

### Beispiele:

Alle Mengenangaben sind, soweit nicht anders vermerkt, Gewichtsteile. Alle Bezeichnungen, sind, soweit nicht anders vermerkt, INCI-Bezeichnungen.

Es wurden Haarreinigungsmittel folgender Zusammensetzung hergestellt:

| | **E1** | **V1** | **E2** | **V2** |
|---|---|---|---|---|
| Carbomer - | - | - | 0,6 | 0,6 |
| Sodium Laureth Sulfate 70%ig | 12,0 | 12,0 | 12,0 | 12,0 |
| Coco-Betaine | 8,0 | 8,0 | 8,0 | 8,0 |
| Dimethicone, 0,65 mm²s⁻¹ | 4,0 | - | - | - |
| Dimethicone 1,0 mm²s⁻¹ | - | - | 4,0 | - |
| hochdisperse Kieselsäure d₅₀= 7 nm | 0,35 | - | 0,35 | - |
| Guar Hydroxypropyltrimonium Chloride | 0,1 | 0,1 | 0,1 | 0,1 |
| Panthenol | 0,02 | 0,02 | 0,02 | 0,02 |
| Euperlan PK 3000 AM* | 2,0 | 2,0 | 2,0 | 2,0 |
| Farbstoff | 0,002 | 0,002 | 0,002 | 0,002 |
| Parfum | 0,4 | 0,4 | 0,4 | 0,4 |
| Citric Acid | 0,2 | 0,2 | 0,2 | 0,2 |
| Sodium Benzoate | 0,5 | 0,5 | - | - |
| DMDM Hydantoin | 0,3 | 0,3 | 0,3 | 0,3 |
| Sodium Methylparabene | 0,3 | 0,3 | 0,3 | 0,3 |
| PEG-40 Hydrogenated Castor | Oil0,25 | 0,25 | 0,25 | 0,25 |
| Sodium Hydroxide | - | - | 0,6 | 0,6 |
| Wasser | ad 100 | ad 100 | ad 100 | ad 100 |
| | | | | |

| Viskosität | | | | |
|---|---|---|---|---|
| (Brookfield 20 U/min, 25°C, DV2) | 4000 | 4000 | 4000 | 4000 |
| pH-Wert | 5,2 | 5,2 | 6,6 | 6,7 |

| | | | | |
|---|---|---|---|---|
| * Perlglanzmittel; INCI-Bezeichnung: Glycol Distearate (and) Laureth-4 (and) Cocamidopropyl Betaine | | | | |

Die Formulierungen V1 und E1 bzw. V2 und E2 wurden im Halbseitentest auf den Köpfen von 10 Probanden getestet. Dazu wurden 20g der jeweiligen Reinigungsmittelzusammensetzung jeweils auf die Fasern an einer Seite des Kopfes der Probanden aufgetragen. Nach einer Einwirkungszeit von 2 min. wurden die Haare gründlich gespült. Nach jeder Wäsche und nach jeweils 3-, 6-, 12- und 18-maliger Haarwäsche wurde das Haar von 3 Friseuren visuell an beiden Seiten beurteilt.

Dabei wurde bei den erfindungsgemäßen Zusammensetzungen E1 bzw. E2 im Vergleich zu den Rezepturen V1 bzw. V2 immer eine signifikante Verbesserung beobachtet (deutlich mehr Fülle der Frisur, mehr Volumen im Haar).

## Patentansprüche

1. Haarreinigungsmittel, enthaltend
a) mindestens ein Silikon der Formel I
(CH₃)₃Si-[O-(CH₃)₂Si]ₓ-O-Si(CH₃)₃ (I),
in der x für eine Zahl von 0 bis 10, vorzugsweise 0, 1 oder 2 steht,
b) mindestens ein Metalloxid und
c) anionische, nichtionische, amphotere und/oder zwitterionische Tenside,
wobei das Gewichtsverhältnis von Silikon(en) zu Metalloxid(en) [a] zu b)] 7 : 1 bis 100 : 1, vorzugsweise 8 : 1 bis 50 : 1, besonders bevorzugt 9 : 1 bis 25 : 1 und insbesondere 10 : 1 bis 20 : 1 beträgt und
wobei die Partikelgröße der Metalloxide zwischen 2 und 100 nm, vorzugsweise zwischen 3 und 80 nm, besonders bevorzugt zwischen 4 und 60 nm und insbesondere zwischen 5 und 50 nm liegt.

2. Mittel nach Anspruch 1, **dadurch gekennzeichnet, daß** es, bezogen auf das Gewicht des Mittels, 0,5 bis 20 Gew.%, vorzugsweise 1 bis 15Gew.%, besonders bevorzugt 2,5 bis 10 Gew.% und insbesondere 3 bis 7 Gew.% Silikon(e) enthält.

3. Mittel nach einem der Ansprüche 1 oder 2, **dadurch gekennzeichnet, daß** es, bezogen auf das Gewicht des Mittels, 0,01 bis 5 Gew.%, vorzugsweise 0,1 bis 2,5 Gew.%, besonders bevorzugt 0,25 bis 1,5 Gew.% und insbesondere 0,3 bis 0,5 Gew.% Metalloxid(e) enthält.

4. Mittel nach einem der Ansprüche 1 bis 3, **dadurch gekennzeichnet, daß** das Metalloxid ausgewählt ist aus Siliciumdioxid, Calciumoxid, Magnesiumoxid und Aluminiumoxid.

5. Mittel nach einem der Ansprüche 1 bis 4, **dadurch gekennzeichnet, daß** das Metalloxid eine Stampfdichte gemäß DIN/ISO 787/11 von 10 - 400 g/l, vorzugsweise von 20 bis 300 g/l, besonders bevorzugt von 30 bis 200 g/l und insbesondere von 40 bis 120 g/l aufweist.

6. Mittel nach einem der Ansprüche 1 bis 5, **dadurch gekennzeichnet, daß** das Metalloxid eine BET-Oberfläche gemäß DIN 66131 von 50 bis 800 m²/g, vorzugsweise von 75 bis 600 m²/g, besonders bevorzugt von 100 bis 500 m²/g, und insbesondere von 130 bis 450 m²/g aufweist.

7. Mittel nach einem der Ansprüche 1 bis 6, **dadurch gekennzeichnet, daß** es ein hydrophob modifiziertes Metalloxid enthält.

8. Mittel nach einem der Ansprüche 1 bis 7, **dadurch gekennzeichnet, daß** es eine Dispersion des Metalloxids b) im Silikon a) enthält.

9. Mittel nach Anspruch 8, **dadurch gekennzeichnet, daß** die Dispersion von Metalloxid in Silikon, bezogen auf das Gewicht der Dispersion, 2 bis 20 Gew.%, vorzugsweise 4 bis 15 Gew.%, besonders bevorzugt 5 bis 12 Gew.% und insbesondere 6 bis 10 Gew.% Metalloxid(e) enthält.

10. Mittel nach einem der Ansprüche 1 bis 9, **dadurch gekennzeichnet, daß** es zusätzlich (eine) Polyhydroxyverbindung(en) enhält, die vorzugsweise ausgewählt ist/sind aus Ethylenglycol, Propylenglycol, Polyethylenglycol, Polypropylenglycol, Glycerin, Pentaerythrit, Sorbit, Mannit, Xylit und ihren Mischungen.

11. Mittel nach einem der Ansprüche 1 bis 10, **dadurch gekennzeichnet, daß** es zusätzlich mindestens einen Stoff aus der Gruppe der Vitamine, Provitamine und Vitaminvorstufen sowie deren Derivate enthält, wobei Vitamine, Pro-Vitamine und Vitaminvorstufen bevorzugt sind, die den Gruppen A, B, C, E, F und H zugeordnet werden.

12. Mittel nach einem der Ansprüche 1 bis 11, **dadurch gekennzeichnet, daß** es zusätzlich ein aminofunktionelles Silikon der Formel (II)
R'ₐG₃₋ₐ-Si(OSiG ₂)ₙ-(OSiG _{b}R'_{2- b})ₘ-O-SiG₃₋ₐ-R'ₐ (II),
enthält, worin bedeutet:
- G ist-H, eine Phenylgruppe, -OH, -O-CH₃, -CH₃, -CH₂CH₃, - CH₂CH₂CH₃, -CH(CH₃)₂, -CH₂CH₂CH₂H₃, -CH₂CH(CH₃)₂, - CH(CH₃)CH₂CH₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
○ -N(R")-CH₂-CH ₂- N(R")₂
○ -N(R")₂
○ -N⁺(R")₃A⁻
○ -N⁺H(R")₂ A⁻
○ -N⁺H₂(R")A⁻
○ -N(R")-CH₂-CH₂-N⁺R"H₂A⁻,
wobei jedes R" für gleiche oder verschiedene Reste aus der Gruppe -H, - Phenyl, -Benzyl, der C₁₋₂₀-Alkylreste, vorzugsweise -CH₃, -CH₂CH₃, - CH₂CH₂CH₃, -CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

13. Verfahren zur Herstellung von Haarreinigungsmitteln, **dadurch gekennzeichnet, daß** mindestens ein Metalloxid in mindestens einem Silikon der Formel I
(CH₃)₃Si-[O-(CH₃)₂Si]ₓ-O-Si(CH₃)₃ (I),
in der x für eine Zahl von 0 bis 10, vorzugsweise 0, 1 oder 2 steht, dispergiert und diese Dispersion mit weiteren Inhaltstoffen von Haarreinigungsmitteln vermischt wird.

14. Verwendung einer Zubereitung nach einem der Ansprüche 1 bis 12 zur Reinigung von Haut und Haar.

15. Verfahren zur nicht-therapeutischen Behandlung von Haut oder Haar, bei dem eine Zubereitung gemäß einem der Ansprüche 1 bis 12 auf die Haut und/oder das Haar aufgetragen wird, wobei die Zubereitung nach einer Einwirkzeit von 0 bis 45 Minuten wieder ausgespült wird.

## Claims

1. A hair cleaning agent, containing
a) at least one silicone of formula I
(CH₃)₃Si-[O-(CH₃)₂Si]ₓ-O-Si(CH₃)₃ (I),
wherein x represents a number from 0 to 10, preferably 0, 1 or 2,
b) at least one metal oxide and
c) anionic, non-ionic, amphoteric and/or zwitterionic surfactants, wherein the weight ratio of silicone(s) to metal oxide(s) [a) to b)] is from 7:1 to 100:1, preferably 8:1 to 50:1, more preferably 9:1 to 25:1 and in particular 10:1 to 20:1 and
wherein the particle size of the metal oxides is between 2 and 100 nm, preferably between 3 and 80 nm, more preferably between 4 and 60 nm and in particular between 5 and 50 nm.

2. The agent according to claim 1, **characterized in that** it contains 0.5 to 20% by weight, preferably 1 to 15% by weight, more preferably 2.5 to 10% by weight, and in particular 3 to 7% by weight of silicone(s), based on the weight of the agent.

3. The agent according to any of claims 1 or 2, **characterized in that** it contains 0.01 to 5% by weight, preferably 0.1 to 2.5% by weight, more preferably 0.25 to 1.5% by weight, and in particular 0.3 to 0.5% by weight of metal oxide(s), based on the weight of the agent.

4. The agent according to any of claims 1 to 3, **characterized in that** the metal oxide is selected from silicon dioxide, calcium oxide, magnesium oxide and aluminium oxide.

5. The agent according to any of claims 1 to 4, **characterized in that** the metal oxide has a tapped density according to DIN/ISO 787/11 of 10-400 g/L, preferably from 20 to 300 g/L, more preferably from 30 to 200 g/L and in particular from 40 to 120 g/L.

6. The agent according to any of claims 1 to 5, **characterized in that** the metal oxide has a BET surface area according to DIN 66131 from 50 to 800 m²/g, preferably from 75 to 600 m²/g, more preferably from 100 to 500 m²/g, and in particular from 130 to 450 m²/g.

7. The agent according to any of claims 1 to 6, **characterized in that** it contains a hydrophobically modified metal oxide.

8. The agent according to any of claims 1 to 7, **characterized in that** it contains a dispersion of the metal oxide b) in the silicone a).

9. The agent according to claim 8, **characterized in that** the dispersion of metal oxide in silicone, contains 2 to 20% by weight, preferably 4 to 15% by weight, more preferably 5 to 12% by weight and in particular 6 to 10% by weight of metal oxide(s), based on the weight of the dispersion.

10. The agent according to any of claims 1 to 9, **characterized in that** it further contains polyhydroxy compound(s), which is/are preferably selected from ethyleneglycol, propyleneglycol, polyethyleneglycol, polypropyleneglycol, glycerol, pentaerythritol, sorbitol, mannitol, xylitol, and mixtures thereof.

11. The agent according to any of claims 1 to 10, **characterized in that** it further contains at least one substance from the group of vitamins, provitamins, and vitamin precursors as well as their derivatives, wherein vitamins, provitamins and vitamin precursors which are associated with the groups A, B, C, E, F and H, are preferred.

12. The agent according to any of claims 1 to 11, **characterized in that** it further contains an aminofunctional silicone of formula (II)
R'ₐG₃₋ₐ-Si(OSiG₂)ₙ-(OSiG_{b}R'_{2-b})ₘ-O-SiG₃₋ₐ-R'ₐ (II),
wherein it is meant that:
- G is -H, a phenyl group, -OH, -O-CH₃, -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃;
- a represents a number between 0 and 3, in particular 0;
- b represents a number between 0 and 1, in particular 1,
- m and n are numbers, their sum (m + n) is between 1 and 2000, preferably between 50 and 150, wherein n preferably assumes values from 0 to 1999 and in particular from 49 to 149 and m preferably assumes values from 1 to 2000, in particular from 1 to 10,
- R' is a monovalent radical selected from
○ -N(R")-CH₂-CH₂-N(R")₂
○ -N(R")₂
○ -N⁺(R")₃A⁻
○ -N⁺H(R")₂A⁻
○ -N⁺H₂(R")A⁻
○ -N(R")-CH₂-CH₂-N⁺R"H₂A⁻,
wherein each R" represents identical or different radicals from the groups of -H, -phenyl, -benzyl, C₁-₂₀ alkyl radicals, preferably -CH₃, -CH₂CH₃, -CH₂CH₂CH₃, -CH(CH₃)₂, -CH₂CH₂CH₂CH_{3,}, -CH₂CH(CH₃)₂, -CH(CH₃)CH₂CH₃, -C(CH₃)₃ and A represents an anion, which is preferably selected from chloride, bromide, iodide or methosulfate.

13. A method for preparing hair cleaning agents, **characterized in that** at least one metal oxide is dispersed in at least one silicon of formula I
(CH₃)₃Si-[O-(CH₃)₂Si]ₓ-O-Si(CH₃)₃ (I),
wherein x represents a number from 0 to 10, preferably 0, 1 or 2, and this dispersion is mixed with further ingredients of haircleaning agents.

14. The use of a preparation according to any of claims 1 to 12, for cleaning skin and hair.

15. A method for non-therapeutic treatment of skin or hair, in which a preparation according to any of claims 1 to 12 is applied on the skin and/or the hair, wherein the preparation after an exposure time from 0 to 45 minutes is rinsed off again.

## Revendications

1. Agent de nettoyage capillaire contenant
a) au moins un silicone répondant à la formule I
(CH₃)₃Si-[O-(CH₃)₂Si]ₓ-O-Si(CH₃)₃ (I),
dans laquelle x représente un nombre de 0 à10, de préférence 0, 1 ou 2 ;
b) au moins un oxyde métallique ; et
c) des agents tensioactifs anioniques, non ioniques, amphotères et/ou zwitterioniques
dans lequel la proportion du/des silicones à l'oxyde/aux oxyde(s) métallique(s) [a) à b)] s'élève de 7 : 1 à 100 : 1, de préférence de 8 : 1 à 50 : 1, de manière particulièrement préférée de 9 : 1 à 25 : 1 et en particulier de 10 : 1 à 20 : 1, et
dans lequel la granulométrie des oxydes métalliques se situe entre 2 et 100 nm, de préférence entre 3 et 80 nm, de manière particulièrement préférée entre 4 et 60 nm et en particulier entre 5 et 50 nm.

2. Agent selon la revendication 1, **caractérisé en ce qu'**il contient, rapportés au poids de l'agent, un ou plusieurs silicones à concurrence de 0,5 à 20 % en poids, de préférence à concurrence de 1 à 15 % en poids, de manière particulièrement préférée à concurrence de 2,5 à 10 % en poids et en particulier de 3 à 7 % en poids.

3. Agent selon la revendication 1 ou 2, **caractérisé en ce qu'**il contient, rapportés au poids de l'agent, un ou plusieurs oxydes métalliques à concurrence de 0,01 à 5 % en poids, de préférence à concurrence de 0,1 à 2,5 % en poids, de manière particulièrement préférée à concurrence de 0,25 à 1,5 % en poids et en particulier de 0,3 à 0,5 % en poids.

4. Agent selon l'une quelconque des revendications 1 à 3, **caractérisé en ce que** l'oxyde métallique est choisi parmi le dioxyde de silicium, l'oxyde de calcium, l'oxyde de magnésium et l'oxyde d'aluminium.

5. Agent selon l'une quelconque des revendications 1 à 4, **caractérisé en ce que** l'oxyde métallique possède une densité du produit tassé conformément à la norme DIN/ISO 787/11 de 10 à 400 g/l, de préférence de 20 à 300 g/l, de manière particulièrement préférée de 30 à 200 g/l, et en particulier de 40 à 120 g/l.

6. Agent selon l'une quelconque des revendications 1 à 5, **caractérisé en ce que** l'oxyde métallique possède une surface BET conformément à la norme DIN 66131 de 50 à 800 m²/g, de préférence de 75 à 600 m²/g, de manière particulièrement préférée de 100 à 500 m²/g, et en particulier de 130 à 450 m²/g.

7. Agent selon l'une quelconque des revendications 1 à 6, **caractérisé en ce qu'**il contient un oxyde métallique qui a été soumis à une modification hydrophobe.

8. Agent selon l'une quelconque des revendications 1 à 7, **caractérisé en ce qu'**il contient une dispersion de l'oxyde métallique b) dans le silicone a).

9. Agent selon la revendication 8, **caractérisé en ce que** la dispersion de l'oxyde métallique dans le silicone contient, rapportés au poids de la dispersion, un ou plusieurs oxydes métalliques à concurrence de 2 à 20 % en poids, de préférence à concurrence de 4 à 15 % en poids, de manière particulièrement préférée à concurrence de 5 à 12 % en poids et en particulier de 6 à 10 % en poids.

10. Agent selon l'une quelconque des revendications 1 à 9, **caractérisé en ce qu'**il contient en outre un ou plusieurs composés polyhydroxylés qui sont choisis de préférence parmi l'éthylèneglycol, le propylèneglycol, le polyéthylèneglycol, le polypropylèneglycol, le glycérol, le pentaérythritol, le sorbitol, le mannitol, le xylitol et leurs mélanges.

11. Agent selon l'une quelconque des revendications 1 à 10, **caractérisé en ce qu'**il contient en outre au moins une substance du groupe des vitamines, des provitamines et des précurseurs de vitamines et de leurs dérivés, les vitamines, les provitamines et les précurseurs de vitamines qui sont attribuées aux groupes A, B, C, E, F et H étant préférés.

12. Agent selon l'une quelconque des revendications 1 à 11, **caractérisé en ce qu'**il contient en outre un silicone aminofonctionnel répondant à la formule (II)
R'ₐG₃₋ₐ-Si(OSiG₂)ₙ-(OSiG_{b}R'_{2-b})ₘ-O-SiG₃₋ₐ-R'ₐ (II),
dans laquelle
- G représente un atome d'hydrogène, un groupe phényle, un groupe -OH, un groupe -O-CH₃, un groupe -CH₃, un groupe -CH₂CH₃, un groupe -CH₂CH₂CH₃, un groupe -CH(CH₃)₂, un groupe -CH₂CH₂CH₂CH₃, un groupe -CH₂CH(CH₃)₂, un groupe -CH(CH₃)CH₂CH₃, un groupe -C(CH₃)₃ ;
- a représente un nombre entre 0 et 3, en particulier 0 ;
- b représente un nombre entre 0 et 1, en particulier 1 ;
- m et n représentent des nombres dont la somme (m + n) se situe entre 1 et 2000, de préférence entre 50 et 150, n prenant de préférence des valeurs de 0 à 1999 et en particulier de 49 à 149 et m prenant de préférence des valeurs de 1 à 2000, en particulier de 1 à 1 0 ;
- R' représente un radical monovalent choisi parmi
○ un radical -N(R")-CH₂-CH₂-N(R")₂
○ un radical -N(R")₂
○ un radical -N⁺(R")₃A⁻
○ un radical -N⁺H(R")₂A⁻
○ un radical -N⁺H₂(R")A⁻
○ un radical -N(R")-CH₂-CH₂-N⁺R"H₂A⁻ ;
chaque radical R" représentant des radicaux identiques ou différents choisis parmi le groupe comprenant un atome d'hydrogène, un groupe phényle, un groupe benzyle, des radicaux alkyle en C₁-C₂₀, de préférence, un radical -CH₃, un radical -CH₂CH₃, un radical -CH₂CH₂CH₃, un radical -CH(CH₃)₂, un radical -CH₂CH₂CH₂CH₃, un radical -CH₂CH(CH₃)₂, un radical -CH(CH₃)CH₂CH₃, un radical -C(CH₃)₃, et A représente un anion qui est choisi de préférence parmi un chlorure, un bromure, un iodure ou un méthosulfate.

13. Procédé pour la préparation d'agents de nettoyage capillaire, **caractérisé en ce qu'**on met en dispersion au moins un oxyde métallique dans au moins un silicone répondant à la formule I
(CH₃)₃Si-[O-(CH₃)₂Si]ₓ-O-Si(CH₃)₃ (I),
dans laquelle
x représente un nombre de 0 à 10, de préférence 0, 1 ou 2, et on mélange cette dispersion avec d'autres constituants d'agents de nettoyage capillaire.

14. Utilisation d'une préparation selon l'une quelconque des revendications 1 à 12, pour le nettoyage de la peau et des cheveux.

15. Procédé pour le traitement non thérapeutique de la peau ou des cheveux, dans lequel on applique sur la peau et/ou sur les cheveux une préparation selon l'une quelconque des revendications 1 à 12, la préparation étant à nouveau éliminée par rinçage après l'avoir laissé agir pendant un laps de temps de 0 à 45 minutes.
